# EUROPEAN PATENT APPLICATION

(11) **EP 1 754 489 A1**
(43) Date of publication of application: **21.02.2007**
(21) Application number: 05254263.6
(22) Date of filing: 07.07.2005
(51) Int. Cl.: A61K 38/18

(54) **A method of treating vitiligo using synergistic formation**

(71) Applicant: Ramaiah, Abburi, Prof., New Delhi (IN)
(72) Inventor: Ramaiah, Abburi, Prof., New Delhi (IN)
(74) Representative: Strachan, Victoria Jane

(57) **Abstract**

Synergistic therapies for the treatment of vitiligo are provided. The major therapies for the treatment of vitiligo- a pigmentary disorder characterized by patchy depigmentation of skin are Psoralens plus UV-A, steroids, basic fibroblast growth factor (bFGF) peptide lotion or surgical procedures. Psoralens plus UV-A is effective in about 50% of cases, steroids are limitedly effective only in fast spreading cases of vitiligo and often reoccurs on stoppage of treatment. Surgical treatment is the last resort for vitiligo therapy, when all other therapies failed. It is limitedly effective. Basic fibroblast growth factor peptide(s) lotion was developed as a new mode of therapy for the treatment of vitiligo It went through successfully various phases of clinical trials in India and is allowed to be marketed by the drug controller general (India). It is effective in 80% of stable vitiligo and in segmental vitiligo. Here data are provided and demonstrated that combinatorial treatment of vitiligo by local application of bFGF peptide(s) lotion in association with psoralens and UV-A, or steroids or surgical procedures produce synergistic response and that the rate of re-pigmentation increases synergistically and more efficacious results are obtained than with any of them alone. Any combinatorial therapy comprising the local application of bFGF peptide lotion on the vitiligo patch in combination with any other therapy for the treatment of vitiligo appears to act synergistically.

## Description

### FIELD OF INVENTION

This invention relates to a method of treating vitiligo using synergistic formation.

### BACKGROUND OF THE INVENTION

Vitiligo/leucoderma is an acquired depigmentation of skin characterized by patchy loss of pigment that becomes progressive with time. This disorder affects about 1% of the world population. Traditional therapies for vitiligo mainly include photo chemotherapy with topical/oral psoralens followed by exposure to ultra violet A radiation (PUV-A) or topical/oral steroids. PUV-A therapy is perhaps the main stay in the treatment of vitiligo. However only about 50% of cases get repigmentation. More over in a patient in response to PUV-A many vitiligo patches may repigment partially only and the rest of the patches may remain unresponsive to PUV-A therapy even after long duration of treatment. The repigmentation in the above therapies is a result of multiplication of melanocytes, the cells, which produce the pigment melanin in the skin. The multiplication of melanocytes in response to the above therapies occur from the margins of the vitiligo patch or at the pigmented hair follicles and their migration/spread to the vitiligo patch.

Basic fibroblast growth factor (bFGF) also known as FGF2 so named because it contains a high number of basic amino acid residues (Lysine, Arginine and Histidine) is a potent mitogen for a variety of cell types including melanocytes. Both human and bovine bFGF were isolated and the gene expressing this product were sequenced and cloned. In addition bFGF was found to be expressed in a wide variety of tissue types including placnta, keratinocytes, fibroblasts. The bFGF or its agonist peptides were tested on human volunteers in the various phases of clinical trials in India and found to be successful in repigmenting about 80% of volunteers with stable generalised vitiligo and segmental vitiligo. Patents of interest describing bFGF or agonist peptides and the formulation for their penetrations through intact skin include US patent 6,143,723, Australian patent 722626, Indian patents 185613, 186437.

Vitiligo is a pigmentary disorder with patchy loss of skin pigment melanin Puri.N, Mojamdar M, Anew hypothesis for the etiology of vitiligo, Acta Derm. Venerol (Stochhom), 1989, 69,323-327 that deprivation of a mitogen(s) like basic fibroblast growth factor (bFGF) for melanocytes or its decreased level in the skin of vitiligo patients for as at unknown reason could result in the loss of melanin producing cells melanocytes in skin resulting in vitiligo. Basic fibroblast growth factor (bFGF) also known as FGF2 is a potent mitogen for variety of cell types including melanocytes. Both human and bovine bFGF have been isolated and the gene expressing this product have been sequenced and cloned. In addition bFGF has been found to be expressed in a wide variety of tissue types including pituitary, brain and adrenal gland corpusluteum, retina, kidney, placenta and keratinocytes, fibroblasts. The hypothesis that a mitogen like bFGF may be reduced in its levels in vitiligo patch resulting in loss of melanocytes and the pigment melanin in vitiligo skin and recently from others.

### DESCRIPTION OF INVENTION

According to this invention there is provided a method for combinatorial synergistic therapy for treatment of generalized stable vitiligo and segmental vitiligo comprises local application of an effective amount of a composition comprises 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith psoralens and UV-A (PUV-A therapy). The method for combinatorial synergistic therapy for treatment of vitiligo patches not responding to PUV-A therapy comprises local application of an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith continuing the oral intake of psoralens and exposure to UV-A. The method for combinatorial synergistic therapy for treatment of fast spreading vitiligo comprises local application an effective amount of a composition comprises 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith steroid therapy. The method for combinatorial synergistic therapy for treatment of vitiligo comprises local application of an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 along with surgical procedures. The method for combinatorial synergistic therapy for treatment of vitiligo comprises local application of the composition further comprises 10-15% w/w of solvent, 10-40% w/w of glycols, and 10-40% w/w of at least one penetration enhancing agent.

The local application of bFGF peptide(s) in the formulation described in the US patent 6,143,723 is effective in more than 80% of cases of stable generalised vitiligo or segmental vitiligo. It is felt that the speed of repigmentation and even better out come than 80% success rate may be accomplished if it is combined with the traditional therapies like PUV-A therapy for vitiligo. In addition bFGF lotion therapy is not effective to prevent the fast spread of vitiligo and perhaps a synergistic combinatorial therapy may emerge if bFGF lotion therapy is used in combination with the steroid therapy traditionally used in the case of fast spreading vitiligo. Similarly the local application bFGF peptide(s) lotion therapy may be advantageously used with any other therapy including the surgical therapies.

Synergistic combinatorial therapy for treatment of fast spreading vitiligo cases comprising bFGF peptide(s) in the formulation described in the US patent 6,143,723 and steroid therapy.

The bFGF peptide lotion therapy is effective for stable vitiligo or segmental vitiligo but not for fast spreading vitiligo.

Steroid therapy is the only therapy available in the presently known methods of treatment to treat fast spreading vitiligo (4). However prolonged therapy with steroid produce many side effects (5,6). In addition after stoppage of treatment, the disease reoccurs. The mode of.action of steroids in stopping the fast spread of vitiligo and repigmentation was thought to be brought about by its inhibitory effect on production of auto anti bodies to melanocytes which were shown to be responsible for the fast spread of vitiligo macules (7). It was thought therefore possible that the combinatorial therapy for treatment of vitiligo with local application of bFGF peptide(s) lotion along with oral steroids may accomplish dual goals, that is
1. Arrest of past spread of vitiligo
2. Faster repigmentation of vitiligo macules.

### DESCRIPTION OF THE ACCOMPANYING DRAWINGS:

Fig.1(a) is the representation of vitiligo before treatment with topical bFGF peptide lotion.
Fig. 1(b) is the representation of vitiligo after treatment with topical bFGF peptide lotion.
Fig.2(a) is the representation of fast spreading vitiligo before treatment with a combination of steroid and topical bFGF peptide lotion.
Fig.2(b) in the representation of fast spreading vitiligo after treatment with a combination of steroid and topical bFGF peptide lotion.
Fig.3(a) is the representation of the vitiligo before the treatment with a combination of topical bFGF peptide lotion and PUVASOL.
Fig.3(b) is the representation of the vitiligo after the treatment with a combination of topical bFGF peptide lotion and PUVASOL.
Fig.4(a) is the representation of vitiligo before the treatment with topical bFGF peptide lotion and PUVASOL.
Fig.4(b) is the representation of vitiligo after the treatment with topical bFGF peptide lotion and PUVASOL.
Fig.5(a) is the representation of vitiligo resistant for more than a year for PUVA before the treatment with topical peptide lotion and PUVA.
Fig.5(b) is the representation of vitiligo resistant for more than a year for PUVA after the treatment with topical peptide bFGF lotion and PUVA.
Fig.6(a) is the representation of vitiligo resistant for more than a year for PUVA before the treatment with topical peptide bFGF lotion and PUVA.
Fig.6(b) is the representation of vitiligo resistant for more than a year for PUVA after the treatment with topical peptide bFGF lotion and PUVA.
Fig.7(a) is the representation of vitiligo resistant for more than a year for PUVA before the treatment with topical peptide bFGF lotion and PUVA.
Fig.7(b) is the representation of vitiligo resistant for more than a year for PUVA after the treatment with topical peptide bFGF lotion and PUVA.
Fig.8(a) L is the representation of vitiligo after patch grafting but before start of the treatment with bFGF peptide lotion. R is the control.
Fig.8(b) L is the representation of vitiligo after patch grafting and after treatment with bFGF peptide lotion for 2 months. R is the control.

### EXAMPLE: 1

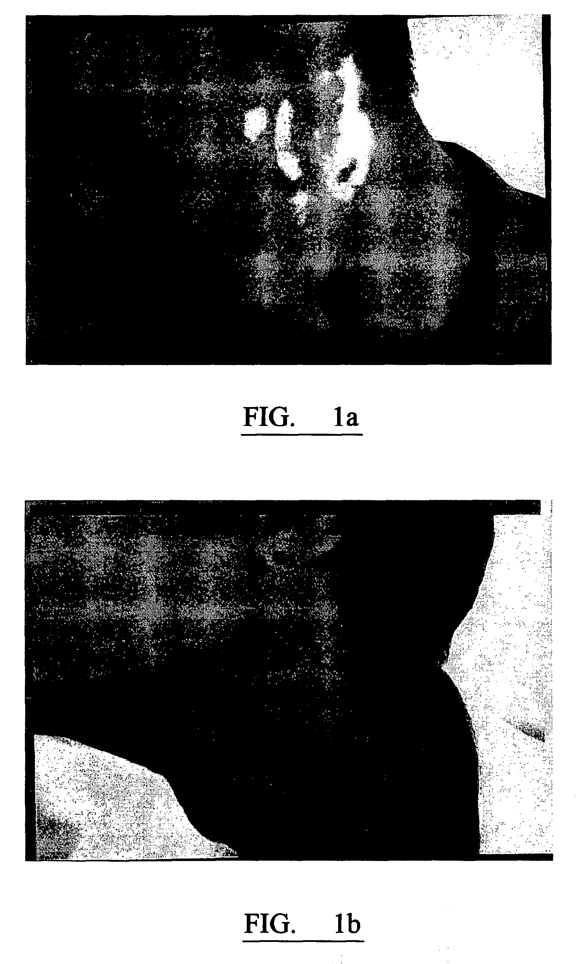

As a typical illustration of efficacy of bFGF peptide(s) in repigmentation of vitiligo patch of human volunteers (Fig.1a, 1b) were depicted. Fig.1a was the size of the vitiligo patch before start of treatment with bFGF peptide lotion and 1b was of the same patch after local application of the bFGF peptide(s) lotion for a period of 6 months. However the above treatment was effective in 80% of stable vitiligo and in segmented vitiligo cases (See Table 1,2 for more details).

Patents of interest describing bFGF or agonist peptides derived from it for use as pigmentary agents include US Patent 6,143,723, Australian Patent No. 722626, Indian Patents 185613, 186437.

The local application of bFGF peptide(s) in the formulation described in the US patent 6,143,723 is effective in more than 80% of cases of stable generalized vitiligo or segmental vitiligo. It is felt that the speed of repigmentation and even better out come than 80% success rate may be accomplished if it is combined with the traditional therapies like PUV-A therapy for vitiligo. In addition bFGF lotion therapy is not effective to prevent the fast spread of vitiligo and perhaps a synergistic combinatorial therapy may emerge if bFGF lotion therapy is used in combination with the steroid therapy traditionally used in the case of fast spreading vitiligo. Similarly the local application bFGF peptide(s) lotion therapy may be advantageously used with any other therapy including the surgical therapies.

### EXAMPLE: 2

As a typical illustration of efficacy of bFGF peptide(s) in repigmentation of vitiligo patch of human volunteers (Fig.1a, 1b) were depicted Fig.1a was the size of the vitiligo patch before start of treatment with bFGF peptide lotion and 1b was of the same patch after local application of the bFGF peptide(s) lotion for a period of 6 months. However the above treatment was effective in 80% of stable vitiligo and in segmental vitiligo cases (See Table-1,2 for more details).

Patents of interest describing bFGF or agonist peptides derived from it for use as pigmentary agents include US Patent 6,143,723, Australian Patent No. 722626, Indian Patents 185613, 186437.

### EXAMPLE:3

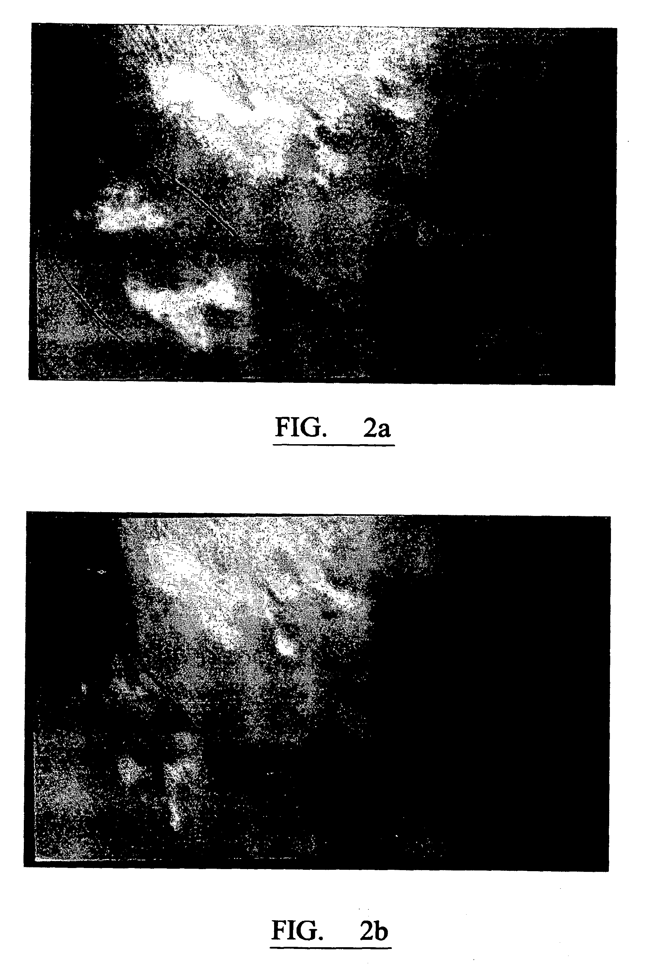

That this happens was shown in a clinical trial on human volunteers. The above twin objectives were accomplished. A typical case of such treatment was shown in Fig.2a, 2b.
Fig.2a describes two vitiligo patches at the start of treatment of oral steroids, while 2b describes the same patches at the end of 3 months of treatment with oral steroids while at the same time bFGF peptide(s) lotion was applied locally once a day on the lower vitiligo patch for the duration of intake of oral steroids. It can be clearly seen that there was faster repigmentation where bFGF peptide(s) lotion was applied as compared to the upper vitiligo patch where there was no application of bFGF peptide lotion. The arrest of the fast spread of the disease was accomplished to the same extent of 84% at the end of 6 months of treatment with oral steroids and the result was not altered by the local application of bFGF peptide(s) lotion (See table 3 for more details).

At the end of 3 months of combinatorial treatment with steroids and local application of bFGF lotion more than 3 fold more patients got pigmented to a marked extent than with steroid alone (See Table 4 for more details). The percentage of volunteers who had moderate and marked rate of repigmentation with combinatorial therapy at the end of three months of treatment is twice than the steroid therapy alone.

Synergistic combinatorial therapy for treatment of stable vitiligo comprising local application of bFGF peptide(s) and oral psoralens plus application of UV-A.

The major or main stay of treatment of stable vitiligo all over the world is at present psoralens and UV-A therapy (8-10). However it is only partially effective (11) and requires long duration of treatment. In addition it has many side effects particularly on liver and the risk of cancer in the Caucasian skin type 11 and 111 population (1213). Therefore there is indeed a need to reduce the
a) duration of the treatment
b) to increase success rate
c) to treat cases non responsive to PUV-A therapy.

The mechanism of action of psoralens and UV-A in repigmentation of vitiligo patches is still an enigma. Psoralens by thenselves are ineffective in treating vitiligo unless followed by exposure of skin to UV-A (14). The absorption maxima of psoralens lie in the 210-330 nm range (15) and the action spectrum of ingested psoralens is probably in the range of 320-335 nm (16). The psoralens induced skin photosensitization involves interchelation of psoralens with the DNA of the cell, which leads eventually to the death of that cell. That this is true in the case of melanocytes also was shown by exposure of melanocytes in culture to PUV-A (17). How then to explain the beneficial effects of PUV-A in increasing the proliferation of melanocytes, their migration to vitiligo patches and thus repigment vitiligo macules? The main target of PUV-A is the epidermis, which receives UV-A radiation. This could kill keratinocytes of the epidermis. The cellular contents of keratinocytes can then leak and stimulate the proliferation of the adjacent melonacytes (18). The cellular contents of keratinocytes include many mitogens to melanocytes in addition to bFGF. That the serum from successfully treated vitiligo patients with PUV-A stimulate the proliferation of melonacytes from normal untreated vitiligo patients was shown in 1989(1).

In addition it was shown that the melonacytes from successfully treated vitiligo patients grow with less generation time than the melanocytes from the untreated normal individuals (1). These results indicated that the combinatorial treatment of vitiligo comprising local application of bFGF peptide(s) lotion with oral psoralens plus UV-A may act synergistically and produce faster repigmentation than with either of them alone.

### EXAMPLE:4

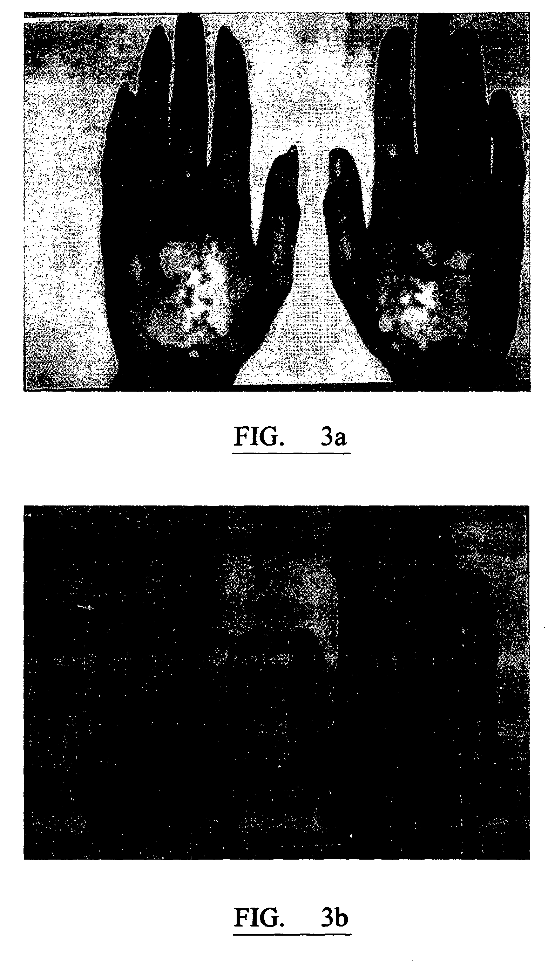

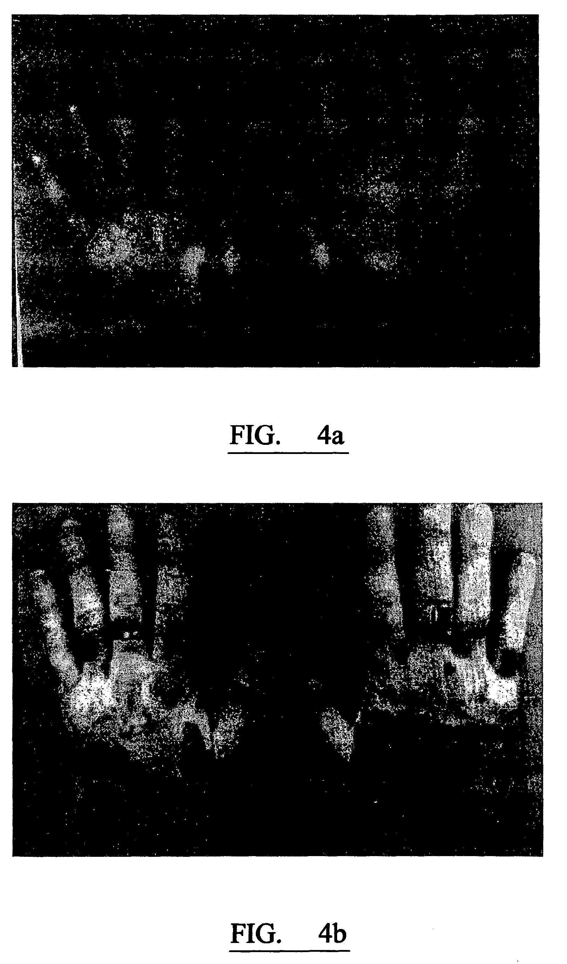

That indeed the combinatorial therapy for treatment of vitiligo comprising local application ofbFGF peptide(s) lotion and PUV-A works synergistically could be seen in the typical pictures shown in Fig.3a, 3b and 4a, 4b. The 3a and 4a are the pictures of two volunteers before the combinatorial treatment, while 3b and 4b are the pictures of the same volunteers respectively at the end of 3 months of combinatorial treatment where the right hand of each voluteer was in addition treated with the local application ofbFGF peptide(s) lotion. As can be seen from the pictures the right hands of both the volunteers treated in addition with the local application of bFGF peptide(s) lotion repigmented many times more than that of the left hands of both the volunteers, which were only treated with the PUVA therapy only. The data presented in table 4 clearly indicate that 7 volunteers treated with combinatorial treatment with bFGF peptide(s) lotion and PUV-A had marked repigmentation in 3 months compared to only one with PUV-A. The percentage of volunteers who had moderate to marked rate of repigmentation with the combinatorial therapy at the end of three months is about twice than the number with PUV-A therapy alone (See table 5 for details).

Synergistic combinatorial therapy for treatment of vitiligo not responding to PUV-A comprising local application of bFGF peptide(s) and PUV-A therapy.

In response to PUV-A therapy, less than 20% of vitiligo patches repigment fully, 30-40% of vitiligo patches repigment partially (11). The vitiligo patches of volunteers, who did not respond PUV-A therapy even after prolonged treatment for a year or more were selected for the combinatorial therapy of local application of bFGF peptide(s) lotion with the continued PUV-A therapy.

### EXAMPLE:5

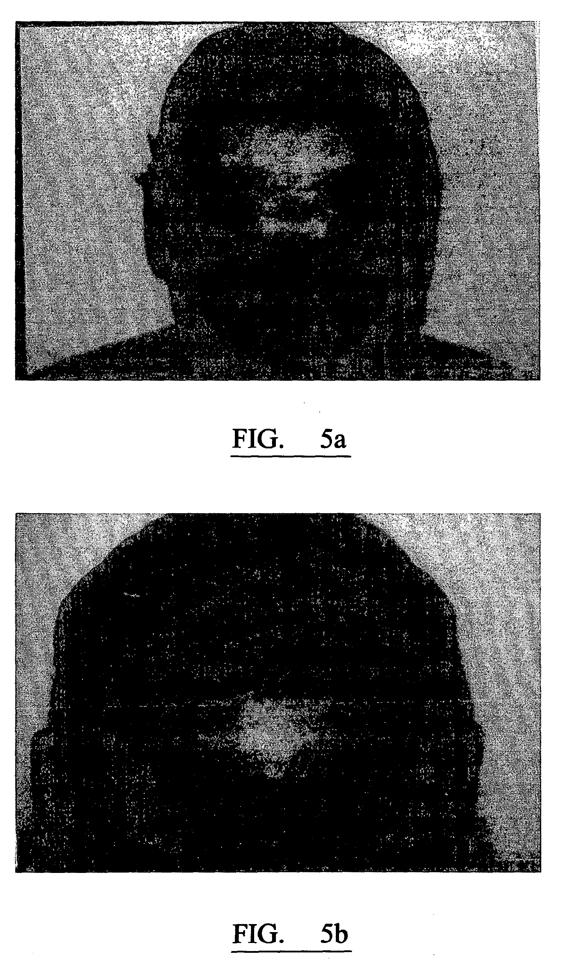

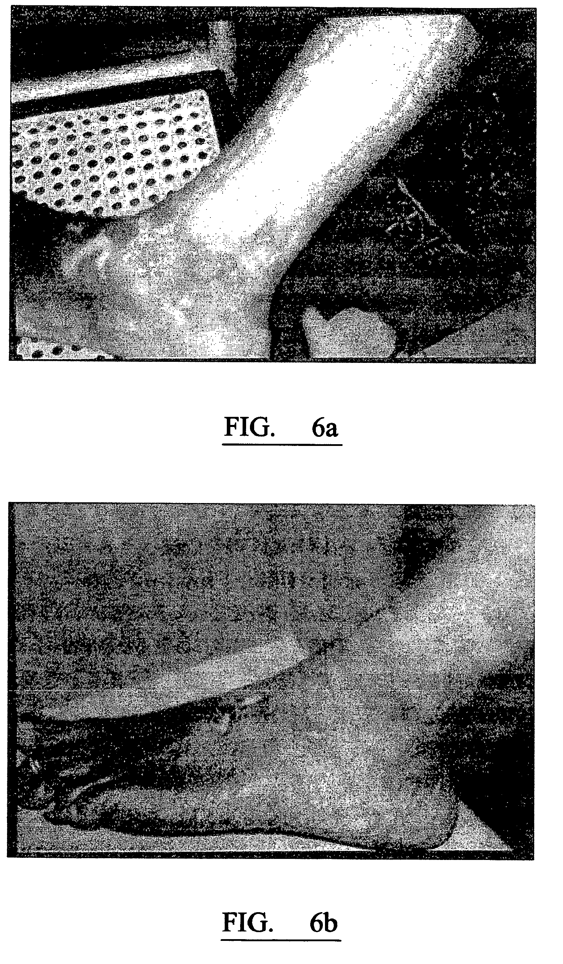

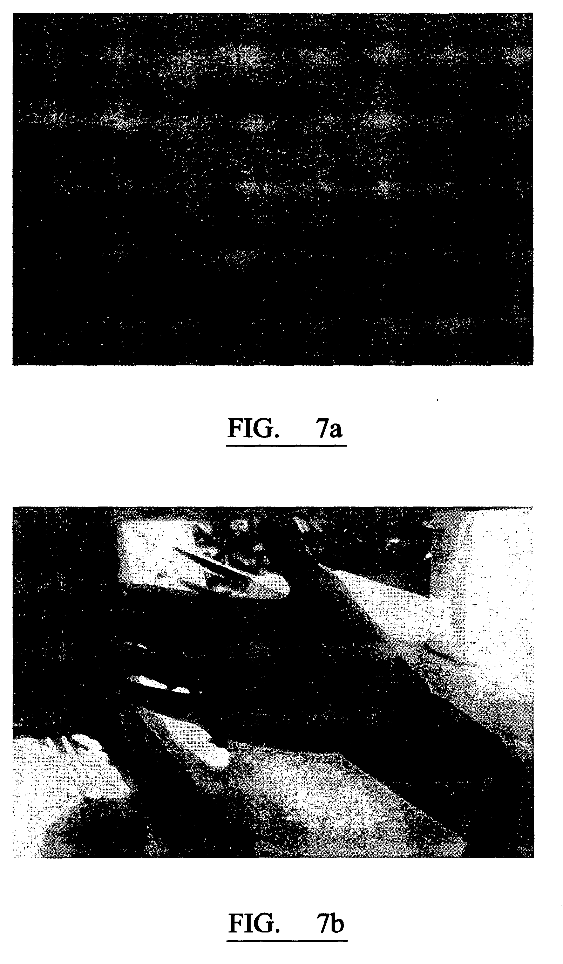

The combinatorial therapy was very successful in repigmenting vitiligo patches with in 3 months which were unresponsive for more than year to PUV-A therapy. The typical results were shown in fig 5a, 5b and 6a, 6b and 7a, 7b. The rate of repigmentation is indeed remarkable in 5a, 5b and 6a,6b. It is also remarkable that the vitiligo patch in the palm which normally never responds to PUV-A therapy even after prolonged duration responded well to combinatorial therapy ofbFGF peptide(s) lotion and PUV-A. The response to combinatorial therapy was shown in more than 90% of cases (See table 6 for details).

The results presented here were not exhaustive. The combinatorial therapy with local application of bFGF peptide(s) in the formulation described in the US patent 6, 143, 723 can be applied to almost any other therapy that are now in the market for the treatment of various types of vitiligo with synergistic effects similar to what was observed in the cases described above. The bFGF peptide(s) in the formulation may be applied locally not only in the form of a lotion but also in any other form like gel/ointment/cream with similar results.

Synergistic combinatorial therapy for the treatment of vitiligo comprising punch graft surgical procedure/or other surgical procedures followed by the local application of bFGF peptides.

Surgical procedures as mentioned earlier are resorted to as the last chance for the treatment of vitiligo patches. They include thrish grafting, expansion of autologus melanocytes in culture and their application on to dermabraded vitiligo patch, autologus expansion of keratinocytes and melaonocytes in culture in the form of a multi cell layered sheets and their application on the dermabraded vitiligo patches, or punch grafting of pigmented autologus skin on to the dermabraded vitiligo patches. In all these cases, after the surgical procedures, the vitiligo patches were surgical bandaged with appropriate antibiotic regimen for the healing of the wounds. The wounds heal with in week or so. Then the local application of the bFGF peptide(s) in the formulation described in the US patent 6,143,723 was done for 3 months. This results in faster repigmentation uniformly.

### EXAMPLE:6

The photographs 8a and 8b describe the synergistic effect on increasing the rate of repigmentation in 8b where the local application of the bFGF peptide(s) in the formulation was done following the punch graft surgical procedure.

**Table - 1**

| **Effect of Local Application of Active Peptide on Repigmentation of all types of Vitiligo Patches of Volunteers** | | | | | |
|---|---|---|---|---|---|
| **Quality of Improvement** | **At 12 weeks** | **% of total** | **End of treatment** | **% of total** | **% significant repigmentation at end of treatment** |
| Marked | 15 | 15.3 | 49 | 50 | 80 |
| Moderate | 31 | 31.6 | 29 | 30 | |
| Minimal | 52 | 53 | 20 | 20 | |
| Nil | 0 | | 0 | | |

**Table - 2**

| **Effect of Local Application of Active Peptide on Repigmentation of patches of Segmental Vitiligo of Volunteers** | | | | | |
|---|---|---|---|---|---|
| **Quality of Improvement** | **At 12 weeks** | **% of total** | **End of treatment** | **% of total** | **% significant repigmentation at end of treatment** |
| Marked | 1 | 4.3 | 16 | 70 | 83 |
| Moderate | 8 | 34.7 | 3 | 13 | |
| Minimal | 14 4 | 60.8 | 4 | 17.4 | |

**Table - 3**

| **Disease Activity of Vitiligo in Patients treat with either OMP of beta methasone or together with local application of active peptide** | | | | |
|---|---|---|---|---|
| **Treatment Regimen** | **No. of patients where the disease was arrested with time** | | | |
| | **At 3 months** | | **At 6 months** | |
| Active Peptide + OMP | 12 | 75% | 14 | 87.5% |
| OMP Alone | 12 | 75% | 14 | 87.5% |

**Table - 4**

| **Effect of oralminipulse of beta methasone and local application of active peptide on repigmentation of vitiligo patches of volunteers with time** | | | | | | |
|---|---|---|---|---|---|---|
| **Treatment Regimen** | **At 3 months** | | | **At 6 months** | | |
| | **Minimal** | **Moderate** | **Marked** | **Minimal** | **Moderate** | **Marked** |
| Active Peptide + OMP | 5 | 5 | 6 | 4 | 4 | 8 |
| OMP Alone | 11 | 3 | 2 | 8 | 4 | 4 |

**Table - 5**

| **Effect of PUV-A Sol and local application of active peptide on repigmentation of vitiligo patches of volunteers with time** | | | | | | |
|---|---|---|---|---|---|---|
| **Treatment Regimen** | **At 3 months** | | | **At 6 months** | | |
| | **Minimal** | **Moderate** | **Marked** | **Minimal** | **Moderate** | **Marked** |
| Active Peptide + PUV-A Sol | 6 | 5 | 7 | 2 | 4 | 12 |
| PUV-A Sol Alone | 11 | 6 | 1 | 7 | 5 | 6 |

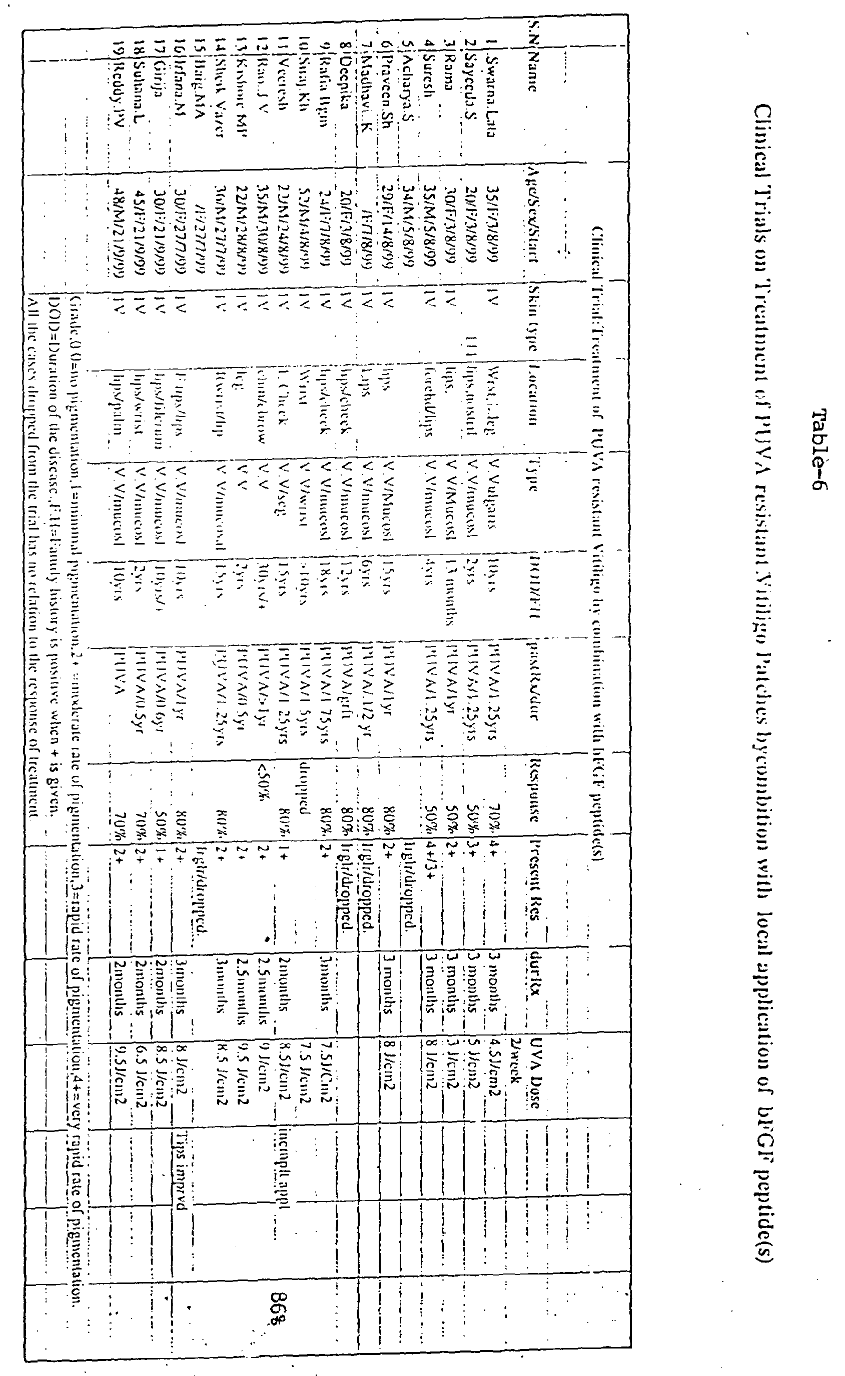

## Claims

1. A method for combinatorial synergistic therapy for treatment of generalized stable vitiligo and segmental vitiligo comprising local application of an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith psoralens and UV-A (PUV-A therapy).

2. A method for combinatorial synergistic therapy for treatment of vitiligo patches not responding to PUV-A therapy comprising local application of an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith continuing the oral intake of psoralens and exposure to UV-A.

3. A method for combinatorial synergistic therapy for treatment of fast spreading vitiligo comprising local application an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith steroid therapy.

4. A method for combinatorial synergistic therapy for treatment of vitiligo comprising local application of an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 along with surgical procedures.

5. The method for combinatorial synergistic therapy for treatment of vitiligo as claimed in claim 1 comprising local application of the composition further comprises 10-15% w/w of solvent, 10-40% w/w of glycols, and 10-40% w/w of at least one penetration enhancing agent.

6. A method for combinatorial synergistic therapy for treatment of vitiligo comprising local application of an effective amount of a composition comprising 0.02 to 5% w/w of at least one peptide selected from a group consisting of bFGF amino acids 106-115 (SEQ ID NO 1), bFGF amino acids 106-120 (SEQ ID NO 5), bFGF amino acids 1-24 (SEQ ID NO 6), SEQ ID:2, SEQ ID:3, cyclic bFGF amino acids 106-115 (SEQ ID NO1), cyclic bFGF amino acids 106-120 (SEQ ID NO5), cyclic bFGF amino acids 1-24 (SEQ ID NO 6), cyclic SEQ ID:2 and cyclic SEQ ID:3 alongwith oral/local application of psoralens/or others that act like psoralens plus UV-A/UV-B, or any other radiation or oral/local steroids/any immuno modulators given orally/locally.

7. A method for combinatorial synergistic therapy for treatment of generalized stable vitiligo and segmental vitiligo as substantially herein described and illustrated in the accompany drawings.
